# EUROPEAN PATENT APPLICATION

(11) **EP 4 005 581 A1**
(43) Date of publication of application: **01.06.2022**
(21) Application number: 21800969.4
(22) Date of filing: 02.02.2021
(51) Int. Cl.: A61K 36/185, A61P 31/14

(54) **HYPERICUM JAPONICUM THUNB. EXTRACT AND APPLICATION THEREOF IN PREPARATION OF ANTI-NOVEL CORONAVIRUS DRUG**

(30) Priority: 17.10.2020 CN 202011106518
(71) Applicant: Sun Yat-Sen University, Guangzhou, Guangdong 510275 (CN); GUANGZHOU INSTITUTE OF RESPIRATORY HEALTH, Guangzhou, Guangdong 510120 (CN)
(72) Inventor: SU, Weiwei, Guangzhou, Guangdong 510275 (CN); YANG,, Guangzhou, Guangdong 510120 (CN); ZHONG, Nanshan, Guangzhou, Guangdong 510120 (CN); MA, Qinhai, Guangzhou, Guangdong 510120 (CN); LI, Runfeng, Guangzhou, Guangdong 510120 (CN); LI, Peibo, Guangzhou, Guangdong 510275 (CN); PENG, Wei, Guangzhou, Guangdong 510275 (CN); WU, Hao, Guangzhou, Guangdong 510275 (CN); SHI, Rui, Guangzhou, Guangdong 510275 (CN); WANG, Yonggang, Guangzhou, Guangdong 510275 (CN)
(74) Representative: Sun, Yiming
(86) International application number: PCT/CN2021/073297
(87) International publication number: WO 2022/077795

(57) **Abstract**

The present invention discloses a herba hyperici japonici extract and application thereof to preparation of an anti-novel coronavirus drug. The extract is prepared by the following method of: preparing a water extract of a herba hyperici japonici medicinal material, loading the water extract onto a macroporous resin column, sequentially carrying out elution by water, 20% ethanol and 60% ethanol, collecting 60% ethanol eluate, and concentrating and recovering ethanol to obtain the herba hyperici japonici extract. By experiments, a result shows that the herba hyperici japonici extract has the obvious inhibiting effect on the cytopathic effect caused by Vero E6 cells infected with novel coronavirus (SARS-CoV-2) within a concentration range of 18.75 to 75µg/mL, and is obviously superior to vincetoxicoside B and quercetin monomeric compounds. The herba hyperici japonici extract is expected to be used for preventing and treating novel coronavirus (SARS-CoV-2) infection.

## Description

### TECHNICAL FIELD

The present invention relates to a herba hyperici japonici extract and a medicinal application thereof.

### BACKGROUND

The 2019 novel coronavirus belongs to a β coronavirus and has an envelope, and the 2019 novel coronavirus particles are of a circle or oval shape, are commonly pleomorphic and have the diameter of 60 to 140nm. The gene characteristics of the 2019 novel coronavirus have the obvious differences from SARS-CoV and MERS-CoV. The 2019 novel coronavirus is spread via respiratory droplets and close contact. Main manifestations of infected and diseased patients are fever, dry cough and fatigue, a few patients are accompanied by symptoms of nasal congestion, runny nose, sore throat, myalgia, diarrhea and the like. Severe patients often suffer from dyspnea and/or hyoxemia one week after incidence, and serious patients can be rapidly progressed to the acute respiratory distress syndrome, sepsis shock, irreformable metabolic acidosis, coagulation disorders, the multiple organ failure and the like. Since the outbreak of the epidemic at the end of 2019, the global death toll has exceeded 1 million. However, currently, there are no specific drugs or vaccines for the virus, and it is mainly depended on improvement of autoimmunity. Traditional Chinese medicine has featured advantages in the prevention and treatment of infectious diseases, and discovery on anti-novel coronavirus Chinese herbal medicinal ingredients has an important effect in resisting the novel coronavirus.

Herba hyperici japonici is also named as hypericum japonicum, has other folk names of Xiaoyuanbaocao (a Chinese herbal medicine of which the leaves look a bit like those of hypericum sampsonii hance, but smaller than those of hypericum sampsonii hance), stellaria alsine, sweetscented basil and the like, and is dry herb of hypericum japomicum thumb. Herba hyperici japonici is originally recorded in "Raw Herbal Property Compilation" written by Kejian He in Qing Dynasty, and is widely distributed in Yangtze River basin and areas south of Yangtze River, and the original plant is mainly produced in Guangdong, Guangxi, Sichuan, Guizhou, Jiangxi and the like. Currently, various chemical components such as chromenes, dipeptides, xanthones, phloroglucinol derivatives, flavonoids and the like have been separated and identified from this plant. The modern pharmacological effect research shows that herba hyperici japonici has various pharmacological effects of inhibiting bacteria, resisting viruses, resisting the liver injury, resisting oxidization, inhibiting cancer cells and the like.

### SUMMARY

The present invention aims to provide a herba hyperici japonici extract, and discloses application thereof to preparation of an anti-novel coronavirus drug.

The herba hyperici japonici extract provided by the present invention is prepared by the following method:
preparing a water extract of a herba hyperici japonici medicinal material, loading the water extract onto a macroporous resin column, sequentially carrying out elution by water, 20% ethanol and 60% ethanol, collecting 60% ethanol eluate, and concentrating and recovering ethanol to obtain the herba hyperici japonici extract.

An elution method preferably includes: loading the water extract onto the macroporous resin column, sequentially carrying out elution by the water and the 20% ethanol of which the volumes are 8 to 12 times of the column volume, and then carrying out elution by the 60% ethanol of which the volume is 3 to 7 times of the column volume. And the elution method most preferably includes: sequentially carrying out elution by the water and the 20% ethanol of which the volumes are 10 times of the column volume, and then carrying out elution by the 60% ethanol of which the volume is 5 times of the column volume.

A preparation method of the water extract of the herba hyperici japonici medicinal material may include: carrying out water decoction and extraction on the herba hyperici japonici medicinal material according to a material-to-liquid ratio of 1:10 for 2 to 3 times for 0.5 to 1 hour, and filtering to obtain the water extract. The extraction times are preferably three times, and the time is preferably 0.5 hour.

By experiments, a result shows that the herba hyperici japonici extract has the obvious inhibiting effect on the cytopathic effect caused by Vero E6 cells infected with novel coronavirus (SARS-CoV-2) within a concentration range of 18.75 to 75µg/mL. The herba hyperici japonici extract is expected to be used for preventing and treating novel coronavirus (SARS-CoV-2) infection.

The herba hyperici japonici extract provided by the present invention mainly contains flavonoid ingredients such as quercitrin, isoquercitrin, vincetoxicoside B, quercetin and the like, by measurement on monomeric compounds, both the vincetoxicoside B and the quercetin have a certain effect of resisting the 2019 novel coronavirus, and the effect of resisting this virus is not detected in the quercitrin and the isoquercitrin. Compared with the effects of the vincetoxicoside B and quercetin monomeric compounds, the effect of the extract is obviously reinforced; it is prompted that the effect of resisting this virus is not detected in the compounds of the quercitrin and the isoquercitrin, but when the quercitrin and the isoquercitrin are used together with the vincetoxicoside B and the quercetin, all the compounds may take some synergistic effects together, so that the activity strength of the vincetoxicoside B and the quercetin is improved.

### BRIEF DESCRIPTION OF THE DRAWINGS

Fig. 1 is an HPLC spectrum of a water extract of herba hyperici japonici;
Fig. 2 is an HPLC spectrum of a water elution part of the water extract of herba hyperici japonici;
Fig. 3 is an HPLC spectrum of a 20% ethanol elution part of the water extract of herba hyperici japonici;
Fig. 4 is an HPLC spectrum of a 60% ethanol elution part of the water extract of herba hyperici japonici;
Fig. 5 is an HPLC spectrum of isoquercitrin;
Fig. 6 is an HPLC spectrum of quercitrin;
Fig. 7 is an HPLC spectrum of vincetoxicoside B;
Fig. 8 is an HPLC spectrum of quercetin;
Fig. 9 is a curve diagram of a result of an anti-novel coronavirus pharmaceutical effect of the vincetoxicoside B;
Fig. 10 is a curve diagram of a result of an anti-novel coronavirus pharmaceutical effect of a herba hyperici japonici extract; and
Fig. 11 is a curve diagram of a novel coronavirus pharmaceutical effect detection result of the quercetin.

### DETAILED DESCRIPTION

The present invention will be further illustrated below in combination with specific embodiments.

### Embodiment 1

1kg of a herba hyperici japonici medicinal material is taken and is subjected to water decoction and extraction for three times, water of which the amount is 10 times of that of the herba hyperici japonici medicinal material is used and the extraction time is 0.5 hour each time, filtering is carried out, the obtained filtrate is naturally cooled, the cooled filtrate is loaded onto a D101 macroporous resin column (containing 1,000g of resin), elution is sequentially carried out by water and 20% ethanol of which the volumes are 10 times of the column volume, then elution is carried out by 60% ethanol of which the volume is 5 times of the column volume, eluate of each part is respectively collected, concentration is carried out, ethanol is recovered, and freeze-drying is carried out to obtain a herba hyperici japonici extract.

Each elution part and each reference substance are respective prepared into solutions with proper concentration (16µg/ml to 60µg/ml), and the solutions are filtered by a 0.45µm filter membrane for later use.

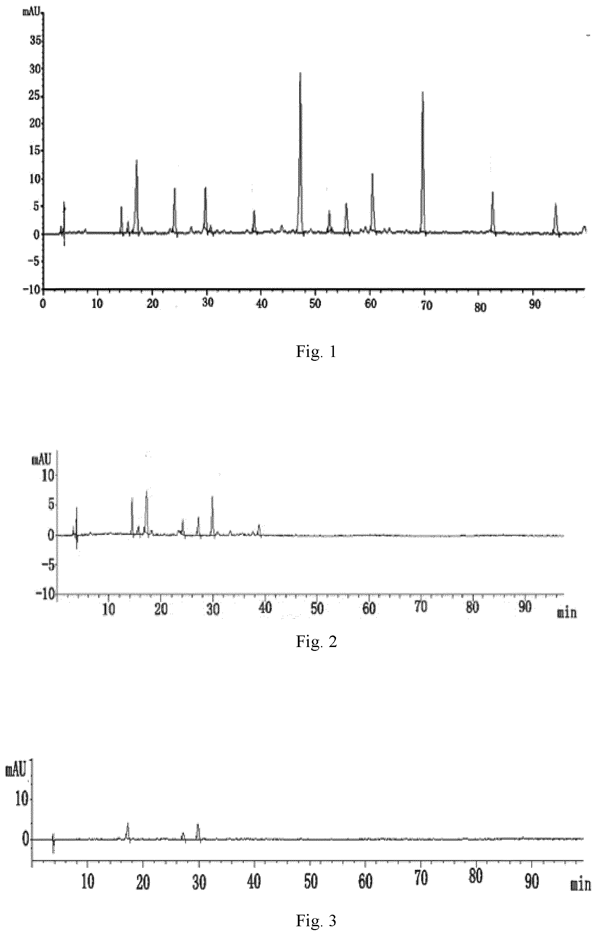

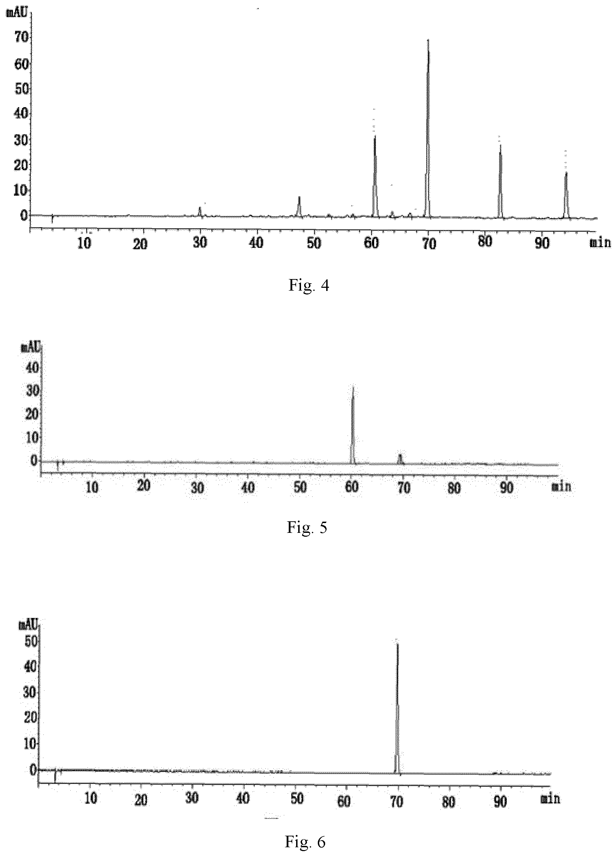

A chromatographic column is an octadecyl bonded silica gel column; a PDA detector is adopted; a mobile phase is an acetonitrile and 0.1% phosphoric acid solution, and linear gradient (concentration of acetonitrile is changed from 5% to 27%) elution is carried out for 0 to 100min; the flow rate is 0.1ml/min; and the detection wavelength is 350nm, and the sample amount is 10µl. The obtained HPLC spectra are as shown in Fig. 1 to Fig. 8.

### Embodiment 2

Experimental Objective: the anti-novel-coronavirus (SARS-CoV-2) effect of a drug is evaluated at a cellular level.

Experimental Materials: the experimental materials include: the herba hyperici japonici extract obtained after each elution part is freeze-dried in Embodiment 1; vincetoxicoside B, which is obtained from a water extract of herba hyperici japonici by column chromatography and has a purity of greater than 95% and the concentration is shown in Table 1; Vero E6 cells, which are preserved in the viral laboratory of the respiratory disease national key laboratory of the Guangzhou Respiratory Health Research Institute; and a virus SARS-CoV-2, which has a titer TCIDso of 10⁻⁶/100 µL and is preserved in the BSL-3 laboratory of the Guangzhou Customs Technology Center (the highly pathogenic microorganism research laboratory of the the respiratory disease national key laboratory) at the temperature of -80°C. The used virus titer is 100TCID₅₀.

Experimental Method: an aseptic 96-well culture plate is adopted, 100µL of Vero E6 cells with the concentration of 2×10⁵ cells/mL is added into each well, and the cells are cultured at 37°C and 5% CO₂ for 24 hours; for the culture plate in experimental groups and a virus control group, 100µL of 100TCID₅₀ virus solution is added per well, and after adsorption is carried out for 2h at the temperature of 37°C in a 5% CO₂ incubator, a cell culture solution in the 96-well culture plate is discarded; tested drugs are diluted into each concentration in Table 1, three complex wells are used for each concentration, and 100µl of drug liquid is added into each well; cell control, blank control (solvent control), and virus control (negative control) are simultaneously set; cells are incubated for 3 to 4 days at the temperature of 37°C in the 5% CO₂ incubator; and the cytopathic effect (CPE) is observed under an optical microscope, and the cytopathic degree is recorded according to the following 6 grades of standards that: "-" represents no cytopathy; "±" represents that cytopathy is smaller than 10%; "+" represents cytopathy is about 25%; "+ +" represents that cytopathy is about 50%; "+ + +" represents cytopathy is about 75%; and "+ + + +" represents that cytopathy is 75% or above. The half effective concentration (IC₅₀) is calculated by adopting a Reed-Muench method or GraphPad Prism5.0. The pharmaceutical effect judgement standard is that: the concentration of inhibiting the CPE of the virus by 50% is regarded as an effective concentration. All the experimental operations above are completed in the BSL-3 laboratory.

**Table 1 Drug Name, Experimental Concentration and Groups**

| Groups | | Concentration (µg/mL) |
|---|---|---|
| Experimental Group 1 | vincetoxicoside B | 60, 30, 15, 7.5 |
| Experimental Group 2 | water eluted extract | 75, 37.5, 18.75, 9.375, 4.6875 |
| Experimental Group 3 | 20% ethanol eluate | 75, 37.5, 18.75, 9.375, 4.6875 |
| Experimental Group 4 | 60% ethanol eluate | 75, 37.5, 18.75, 9.375, 4.6875 |
| Virus Control Group | SARS-CoV-2 virus solution | 100 TCIDso per well |
| Cell Control | culture medium | |

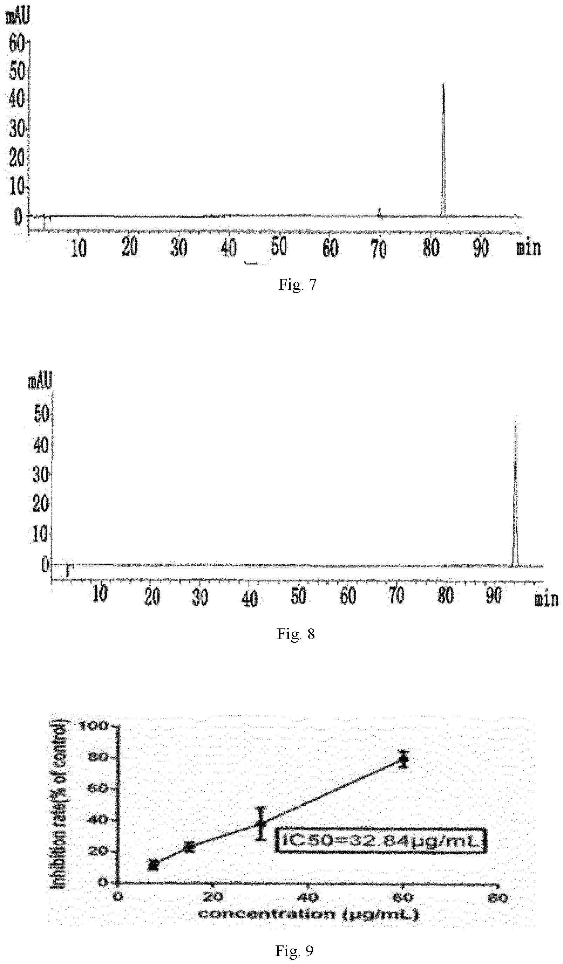

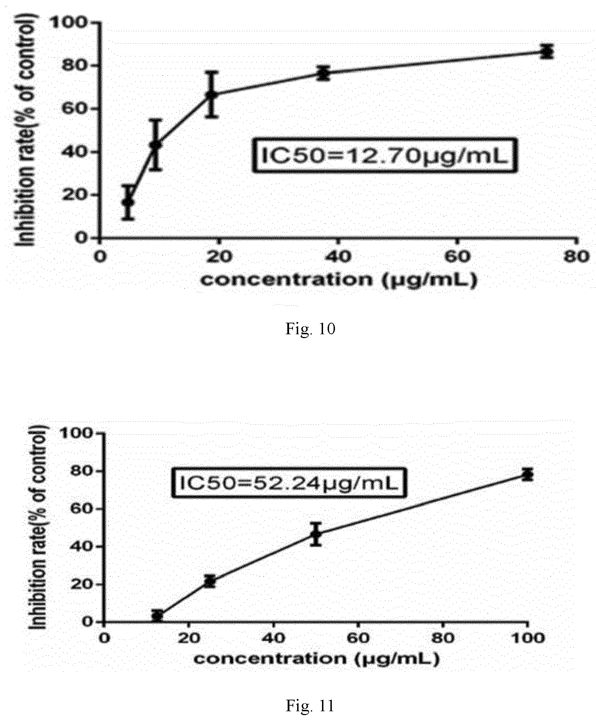

Experimental Results: the cytopathic effect (CPE) is observed, the experimental results are recorded, the half effective concentration (IC₅₀) is calculated by adopting the Reed-Muench method or GraphPad Prism5.0, and the experimental results are shown in Table 2, Table 3, Fig. 9 and Fig. 10.

**Table 2 Results of Anti-novel coronavirus Pharmaceutical Effect of Vincetoxicoside B**

| | |
|---|---|
| Drug Concentration (µg/mL) | Inhibition Rate (%) |
| 60.00 | 80.00±5.00 |
| 30.00 | 38.33±10.41 |
| 15.00 | 23.33±2.89 |
| 7.5 | 11.67±2.89 |

The experimental results show that the vincetoxicoside B has the inhibiting effect on the cytopathic effect caused by the Vero E6 cells infected with novel coronavirus (SARS-CoV-2) under the maximum non-cytotoxic concentration of 60µg/mL.

**Table 3 Results of Anti-novelcoronavirus Pharmaceutical Effect of Herba Hyperici Japonici Extract**

| Drug Concentration (µg/mL) | Inhibition Rate (%) |
|---|---|
| 75.00 | 86.67±2.89 |
| 37.50 | 76.67±2.89 |
| 18.75 | 66.67±10.41 |
| 9.375 | 43.33±11.55 |
| 4.6875 | 16.67±7.64 |

The experimental results show that the herba hyperici japonici extract has the obvious inhibiting effect on the cytopathic effect caused by the Vero E6 cells infected with novel coronavirus (SARS-CoV-2) within a concentration range of 18.75 to 75µg/mL.

The water eluate and 20% ethanol eluate obtained after the water extract of herba hyperici japonici is loaded onto a column are concentrated and freeze-dried, and the obtained extracts have no anti-virus effect.

The results show that the herba hyperici japonici extract obtained by carrying out elution with 60% ethanol after the water extract of herba hyperici japonici is loaded onto the column has the obvious inhibiting effect on the cytopathic effect caused by the Vero E6 cells infected with the novel coronavirus (SARS-CoV-2), and is obviously superior to the ingredient vincetoxicoside B contained in the herba hyperici japonici.

### Embodiment 3

I. Experimental Objective:
During the epidemic emergency, the anti-novel coronavirus (SARS-CoV-2) effect of a drug is evaluated at a cellular level.
II. Experimental Materials:
The experimental materials include: quercitrin, isoquercitrin and quercetin of which the specific dilution rates or concentrations are shown in Table 4; Vero E6 cells, which are preserved in the viral laboratory of the respiratory disease national key laboratory of the Guangzhou Respiratory Health Research Institute; and a virus SARS-CoV-2, which has a titer TCID₅₀ of 10⁻⁶/100 µL and is preserved in the national bio-safety detection key laboratory (P3 laboratory) of the Guangzhou Customs Technology Center Health Inspection and Quarantine Research Institute at the temperature of -80°C. The used virus titer is 100TCID₅₀.
III. Experimental Method:
1. Tested Drugs

**Table 4 Drug Name, Experimental Concentration and Groups**

| Groups | | Concentration (µg/mL) |
|---|---|---|
| Experimental Group 1 | quercitrin | 200, 100, 50, 25 |
| Experimental Group 2 | isoquercitrin | 100, 50, 25, 12.5 |
| Experimental Group 3 | quercetin | 100, 50, 25, 12.5 |
| Virus Control | SARS-CoV-2 virus | 100 TCID₅₀ per well |
| Group | solution | |
| Cell Control | culture medium | |

2. Anti-virus Experiments of Tested Drugs
An aseptic 96-well culture plate is adopted, 100µL of Vero E6 cells with the concentration of 2×10⁵ cells/mL is added into each well, and the cells are cultured at 37°C and 5% CO₂for 24 hours; for the culture plate in experimental groups and a virus control group, 100µL of 100TCID₅₀ virus solution is added per well, and adsorption is carried out for 2h at the temperature of 37°C in a 5% CO₂ incubator; after 2h, a cell culture solution in the 96-well culture plate is discarded; tested drugs are diluted into each concentration in Table 1, three complex wells are used for each concentration, and 100µl of drug liquid is added into each well; cell control, blank control (solvent control), and virus control (negative control) are simultaneously set; cells are incubated for 3 to 4 days at the temperature of 37°C in the 5% CO₂ incubator; and the cytopathic effect (CPE) is observed under an optical microscope, and the cytopathic degree is recorded according to the following 6 grades of standards that: "-" represents no cytopathy; "±" represents that cytopathy is smaller than 10%; "+" represents cytopathy is about 25%; "+ +" represents that cytopathy is about 50%; "+ + +" represents cytopathy is about 75%; and "+ + + +" represents that cytopathy is 75% or above. The half effective concentration (IC₅₀) is calculated by adopting a Reed-Muench method or GraphPad Prism8.0. The pharmaceutical effect judgement standard is that: the concentration of inhibiting the CPE of the virus by 50% is regarded as an effective concentration.
3. Experimental Conditions: all the experimental operations above are completed in a BSL-3 laboratory.
IV. Experimental Results
The cytopathic effect (CPE) is observed, the experimental results are recorded, the half effective concentration (IC₅₀) is calculated by adopting the Reed-Muench method or GraphPad Prism8.0, and the experimental results are shown in Table 5 and Fig. 11.

**Table 5 Results of Anti-novel coronavirus Pharmaceutical Effect of Quercetin**

| Drug Concentration (µg/mL) | Inhibition Rate (%) |
|---|---|
| 100 | 78.33±2.89 |
| 50 | 46.67±5.77 |
| 25 | 21.67±2.89 |
| 12.5 | 3.33±2.89 |

The results show that quercetin has the inhibiting effect on the cytopathic effect caused by the Vero E6 cells infected with the novel coronavirus (SARS-CoV-2) in vitro, but both quercitrin and isoquercitrin do not have the inhibiting effect on the novel coronavirus.

## Claims

1. A herba hyperici japonici extract, **characterized by** being prepared by the following method of: preparing a water extract of a herba hyperici japonici medicinal material, loading the water extract onto a macroporous resin column, sequentially carrying out elution by water, 20% ethanol and 60% ethanol, collecting 60% ethanol eluate, and concentrating and recovering ethanol to obtain the herba hyperici japonici extract.

2. The extract of claim 1, **characterized in that** elution comprises the specific steps of: loading the water extract onto the macroporous resin column, sequentially carrying out elution by the water and the 20% ethanol of which the volumes are 8 to 12 times of the column volume, and then carrying out elution by the 60% ethanol of which the volume is 3 to 7 times of the column volume.

3. The extract of claim 2, **characterized in that** elution comprises the specific steps of: loading the water extract onto the macroporous resin column, sequentially carrying out elution by the water and the 20% ethanol of which the volumes are 10 times of the column volume, and then carrying out elution by the 60% ethanol of which the volume is 5 times of the column volume.

4. The extract of claim 1, **characterized in that** a preparation method of the water extract comprises: carrying out water decoction and extraction on the herba hyperici japonici medicinal material according to a material-to-liquid ratio of 1:10 for 2 to 3 times for 0.5 to 1 hour, and filtering to obtain the water extract.

5. The extract of claim 1, **characterized in that** the preparation method of the extract specifically comprises: carrying out water decoction and extraction on the herba hyperici japonici medicinal material according to a material-to-liquid ratio of 1:10 for 3 times for 0.5 hour, and carrying out filtering and natural cooling; and loading the water extract onto the macroporous resin column, sequentially carrying out elution by the water and the 20% ethanol of which the volumes are 10 times of the column volume, and then carrying out elution by the 60% ethanol of which the volume is 5 times of the column volumm, collecting the 60% ethanol eluate, and concentrating and recovering ethanol to obtain the herba hyperici japonici extract.

6. Application of the extract of any one of claims 1 to 5 to preparation of an anti-2019 novel coronavirus drug.
